# EUROPEAN PATENT APPLICATION

(11) **EP 2 149 367 A1**
(43) Date of publication of application: **03.02.2010**
(21) Application number: 09166432.6
(22) Date of filing: 27.07.2009
(51) Int. Cl.: A61K 8/97, A61K 8/67, A61K 8/365, A61K 8/46, A61K 8/34, A61K 8/65, A61Q 3/02

(54) **Cosmetic composition for nails and related method of application**

(30) Priority: 29.07.2008 IT MI20081391
(71) Applicant: Trasdeco International S.r.l., 20090 Assago (IT)
(72) Inventor: Lenzo, Maurizio, 20090, Trezzano sul Naviglio (Milan) (IT)
(74) Representative: Martegani, Franco

(57) **Abstract**

The following invention refers to a cosmetic composition for finger and toe nails comprising the following ingredients: field horsetail, soy lecithin, vitamin A, alpha-alpha, rhatany, zea mais, keratin, mandelic acid, methylsulfonylmethane and glycerol as well as to a cosmetic patch comprising such composition.

## Description

The present invention regards a cosmetic composition for nails and the related method of application. In particular, the present invention regards a cosmetic composition for nails applicable by means of an adhesive patch.

Over the years, the practice of aesthetic treatments, especially those regarding finger and toe nails, has seen a considerably ever-growing popularity in use both for women and men.

At the same time, alongside this practice, various types of cosmetic products have been developed which are capable of providing various types of effects on the nails and on the skin surrounding the nails.

The products available in the market have very specific effects. For example, there are products in form of nail polish, creams or oils which have a good reinforcement action for the nails, but which are not efficient at curing cuticles; on the other hand, other products are capable of efficiently hydrating the skin around the nails or maintaining the cuticles clean, but they have no effect as regards the nails.

A disadvantage of the products known from the prior art is therefore that of requiring the combined use of different products in such a manner to ensure a complete cosmetic treatment for the finger or toe nails and the treatment of the surrounding skin, with the consequent extension of the time to be devoted to treatment and increase of the related costs. Furthermore, application of products having different specific effects is not always possible. For example, the application of a reinforcement nail polish, which serves as a protective coating, makes the subsequent application of other products inefficient. Therefore, the application of a nail polish for reinforcing the nails cannot be followed by any other type of treatment.

A further disadvantage of cosmetic products for nails known from the prior art is linked to the presence of oil substances (for example creams) or solvents (for example polishes). The presence of this ingredient type requires avoiding contact between nails and the objects until the application is completed in order to prevent the same treatment from becoming inefficient and/or dirtying possibly handled objects with such substances.

Furthermore, also known from the prior art, are cosmetic or curative patches for nails. However, cosmetic patches essentially serve to repair physical damages on the nail (reconstruction), while the curative patches are used to cure specific pathologies of the nail or of the surrounding skin by means of pharmaceutical active ingredients.

Object of the present invention is that of overcoming the drawbacks observed on the prior art.

The first object of the present invention is a cosmetic composition for finger and toe nails comprising the following ingredients: field horsetail, soy lecithin, vitamin A, alpha-alpha, rhatany, zea mais, keratin, mandelic acid, methylsulfonylmethane and glycerol.

The special selection of ingredients distinguishing the composition of the present invention allows simultaneously obtaining various benefits for the finger or toe nails and for the surrounding skin by applying a single product.

In a particular manner, alongside reinforcing, nourishing and preserving the nail, the application of the cosmetic composition of the invention also performs a hydrating action on the surrounding skin. The cosmetic composition according to the invention also acts on the cuticles, keeping the nail contour clean.

In a preferred embodiment, the composition of the present invention has the following ingredient percentage composition by weight:
field horsetail (FE): from 10 to 25%, preferably from 15 to 20%;
soy lecithin(FE): from 10 to 25%; preferably from 15 to 20%;
vitamin A: from 3 to 15%, preferably from 5 to 10%;
alpha-alpha (FE): from 3 to 15%; preferably from 5 to 10%;
rhatany (FE): from 1 to 10%; preferably from 3 to 7%;
zea mais (CE): from 1 to 10%; preferably from 3 to 7%;
keratin: from 0.5 to 6%; preferably from 1 to 4%;
mandelic acid: from 0.5 to 6%; preferably from 1 to 4%;
methylsulfonylmethane: from 0.5 to 6%; preferably from 1 to 4%;
glycerol: from 20 to 50%; preferably from 25 to 40%.

Furthermore, the composition may also contain other ingredients made up of additives typically used in the cosmetic composition, such as surfactants, emulsifiers, stabilisers, perfumes, etc.

The ingredients of the composition according to the present invention are used in the forms typically available for use in preparing cosmetic products. In particular, the vegetable components are used in form of fluid extracts (FE) or crude extracts (CE).

The composition according to the present invention can be prepared starting from different ingredients using techniques known to a man skilled in the art and with the help of equipment conventionally used in the cosmetic preparation industry.

A second object of the present invention is a cosmetic patch comprising a substrate, an adhesive compound and a cosmetic composition for finger and toe nails, said composition comprising the following ingredients: field horsetail, soy lecithin, vitamin A, alpha-alpha, rhatany, zea mais, keratin, mandelic acid, methylsulfonylmethane and glycerol.

Further characteristics of the cosmetic composition capable of being held in the cosmetic patch are the ones described previously for the cosmetic composition subject of the present invention.

Though the ingredients of the composition subject of the present invention are separately known and used in the production of cosmetic products for nails, the particular combination of ingredients described in the present invention has never been described in a single product.

Furthermore, the applicant surprisingly found out that the cosmetic composition of the present invention can be applied on nails by means of an adhesive patch, which serves as a carrier of the ingredients onto the nails and onto the surrounding skin.

The patch comprising the cosmetic composition can be prepared by adding an adhesive compound on the aforedescribed cosmetic composition and thus spreading the resulting composition (hereinafter also indicated as adhesive cosmetic composition) onto the surface of a support substrate made of fabric or any other suitable material, for example the ones commonly used for making patches.

In order to guarantee a good adhesiveness of the patch onto the zone of application, i.e. onto the surface of the nail and onto the area of the surrounding skin, it is possible to use one or more adhesive compounds to be added to the cosmetic composition. The adhesive compounds are made up of solutions comprising mixtures of acrylic compounds in organic solvents (hereinafter also referred to as adhesive solutions). More preferably, the adhesive cosmetic composition comprises only one adhesive solution.

Preferably, the adhesive solution is present in the cosmetic composition in amounts ranging from 64 to 90%, preferably from 75 to 85%, by weight of adhesive solution with respect to the overall weight of the adhesive cosmetic composition.

A layer made of suitable material (closure layer) is applied on the surface of the patch on which the adhesive cosmetic composition is present, such layer being removable before the application, in order to preserve the composition up to the moment of use in cosmetic treatment.

A further object of the present invention is a method for applying a cosmetic composition on finger and/or toe nails, comprising the following steps:
- applying, on each nail to be treated and on the surrounding skin, a cosmetic patch comprising a cosmetic composition comprising the following ingredients: field horsetail, soy lecithin, vitamin A, alpha-alpha, rhatany, zea mais, keratin, mandelic acid, methylsulfonylmethane and glycerol;
- maintaining the application of the patch on each nail for a minimum period of at least 6 hours.

The cosmetic patch may comprise cosmetic compositions having the characteristics described previously.

In the method of application according to the present invention, the cosmetic patch is applied on each nail to be treated and on the surrounding skin, onto which it adheres perfectly fitting onto the shape of the zone intended to be treated.

The beneficial action of the cosmetic composition occurs through direct contact of the ingredients of the composition with the nail and with the surrounding skin. The action extends for the period of time in which the application on the patch is maintained on the zone subjected to treatment. Preferably, the patch is applied for a period ranging from approximately 6 to 8 hours.

Preferably, the patch is applied during a rest phase, preferably during the night, in that the human organism is more receptive concerning the action of the ingredients of the cosmetic composition during night hours. Furthermore, during the night rest hours, external interference factors (such as for example movements and/or peeling off due to interactions with surfaces, objects, etc) which can reduce the efficiency of the application do not occur.

The beneficial effects of the cosmetic composition according to the present invention can already be noticed after the first 2-3 applications.

The cosmetic composition for nails according to the present invention thus allows overcoming the drawbacks arising from the use of cosmetic products of the prior art.

The cosmetic composition of the invention is capable of reinforcing, nourishing and preserving the nails. Furthermore, the cosmetic composition of the invention efficiently hydrates the skin surrounding the nails and acts on the cuticles, maintaining the nail contour clean.

In particular, the cosmetic composition according to the invention allows obtaining all the abovementioned benefits by applying a single product, avoiding the use of several products.

Furthermore, given that its application on the nails occurs by means of an adhesive patch, which insulates the zone under treatment from the external environment, during the cosmetic treatment it is possible to handle objects without dirtying them with oil substances or solvents and without reducing the efficiency of the treatment.

The following embodiments of the present invention are provided merely for illustrative purposes and should not be deemed restrictive in the scope of protection defined by the attached claims.

### EXAMPLE 1

The cosmetic composition was prepared having the following percentage composition:

| | |
|---|---|
| field horsetail (FE): | 16%; |
| soy lecithin (FE): | 16%; |
| vitamin A: | 9%; |
| alpha-alpha (FE): | 9%; |
| rhatany (FE): | 5%; |
| zea mais (CE): | 5%; |
| keratin: | 2%; |
| mandelic acid: | 2%; |
| methylsulfonylmethane: | 2%; |
| glycerol: | 34%. |

Then, an adhesive solution comprising a mixture of acrylic compounds in organic solvents was added to the composition. The adhesive solution added is available in the market by the name Duro-Tak (National Starch and Chemical Co.).

Said solution was added to the cosmetic composition in such an amount that the concentration of the adhesive solution in the resulting adhesive cosmetic composition was equivalent to 85% by weight.

The composition proved to have a good adhesiveness against the substrate of the patch. Equally good was the adhesiveness of the patch against the zone under treatment.

Regular application of the cosmetic patch onto the finger and toe nails generated a reinforcing, hydrating, nutritive and preserving effect, analogous or stronger with respect to the one obtainable by means of cosmetic products known from the prior art.

### EXAMPLE 2

A second composition was prepared starting from the composition of example 1 by adding two adhesive solutions, made up of two solutions comprising two different mixtures of acrylic compounds in organic solvents. The solutions used are available in the market by the commercial names of Duro-Tak (National Starch and Chemical Co.).

The first solution was added in such an amount that the concentration of the adhesive solution in the resulting adhesive cosmetic composition was equivalent to 55% by weight.

The second solution was added in such an amount that the concentration of the adhesive solution in the resulting adhesive cosmetic composition was equivalent to 30% by weight.

The adhesiveness of the composition onto the substrate of the patch and that of the patch onto the zone under treatment was lower with respect to the one observed in example 1.

## Claims

1. Cosmetic composition for finger and toe nails comprising the following ingredients: field horsetail, soy lecithin, vitamin A, alpha-alpha, rhatany, zea mais, keratin, mandelic acid, methylsulfonylmethane and glycerol.

2. Cosmetic composition according to claim 1 comprising the following ingredient composition percentage by weight with respect to the total weight of the composition:
field horsetail (FE): from 10 to 25%, preferably from 15 to 20%;
soy lecithin (FE): from 10 to 25%; preferably from 15 to 20%;
vitamin A: from 3 to 15%, preferably from 5 to 10%;
alpha-alpha (FE): from 3 to 15%; preferably from 5 to 10%;
rhatany (FE): from 1 to 10%; preferably from 3 to 7%;
zea mais (CE): from 1 to 10%; preferably from 3 to 7%;
keratin: from 0.5 to 6%; preferably from 1 to 4%;
mandelic acid: from 0.5 to 6%; preferably from 1 to 4%;
methylsulfonylmethane: from 0.5 to 6%; preferably from 1 to 4%;
glycerol: from 20 to 50%; preferably from 25 to 40%.

3. Cosmetic composition according to claim 1 comprising the following ingredient composition percentage by weight with respect to the total weight of the composition:
| | |
|---|---|
| field horsetail (FE): | 16%; |
| soy lecithin (FE): | 16%; |
| vitamin A: | 9%; |
| alpha-alpha (FE): | 9%; |
| rhatany (FE): | 5%; |
| zea mais (CE): | 5%; |
| keratin: | 5%; |
| mandelic acid: | 2%; |
| methylsulfonylmethane: | 2%; |
| glycerol: | 34%. |

4. Cosmetic composition according to any one of claims 1-3 further comprising one or more additives for cosmetics.

5. Cosmetic patch comprising a support substrate, an adhesive compound and a cosmetic compound for nails as defined in any one of claims 1-4.

6. Cosmetic patch according to claim 5 wherein the cosmetic adhesive is at least one adhesive solution comprising a mixture of acrylic compounds in organic solvents, preferably the adhesive compound is a single adhesive solution.

7. Cosmetic patch according to claim 5 or 6 wherein the adhesive compound is present in an amount variable from 64 to 90%, preferably from 75 to 85%, by weight with respect to the overall weight of the adhesive cosmetic composition.

8. Cosmetic patch according to any one of claims 5-7 further comprising a removable closure substrate.

9. Method for applying a cosmetic composition on finger and/or toe nails, comprising the following steps:
- applying, on each nail to be treated and on the surrounding skin, a cosmetic patch comprising a cosmetic composition comprising the following ingredients: field horsetail, soy lecithin, vitamin A, alpha-alpha, rhatany, zea mais, keratin, mandelic acid, methylsulfonylmethane and glycerol;
- maintaining the application of the patch on each nail for a minimum period of at least 6 hours.

10. Method according to claim 9 wherein the cosmetic patch is applied for a period of time ranging from approximately 6 to 8 hours.

11. Method according to claims 9 or 10 wherein the cosmetic patch is applied during a rest phase, preferably during the night.
